# DEMANDE DE BREVET EUROPEEN

(11) **EP 2 489 374 A1**
(43) Date de publication de la demande: **22.08.2012**
(21) Numéro de dépôt: 12168352.8
(22) Date de dépôt: 06.12.2007
(51) Int. Cl.: A61L 27/20, A61L 27/52, A61K 8/73, A61Q 19/08, A61K 8/02, A61K 8/04

(54) **Gel d'acide hyaluronique pour injection intradermique**

(30) Priorité: 06.12.2006 FR 0610645
(62) Demande divisionnaire de: 07847872.4
(71) Demandeur: Pierre Fabre Dermo-Cosmétique, 92100 Boulogne-Billancourt (FR)
(72) Inventeur: Piron, Estelle, 73540 LA BATHIE (FR); Bogdanowicz, Patrick, 31130 BALMA (FR)
(74) Mandataire: Ahner, Francis

(57) **Abrégé**

Implant injectable par voie sous-cutanée ou intradermique sous la forme d'un hydrogel monophasique comprenant un gel constitué d'acide hyaluronique réticulé et l'un de ses sels physiologiquement acceptables.

## Description

La présente invention concerne des implants injectables par voie sous-cutanée ou intradermique à base d'acide hyaluronique.

L'acide hyaluronique (HA) sous sa forme acide ou sous forme de sel (hyaluronate) est la composante majeure de la matrice extracellulaire. Il est surtout présent dans les tissus conjonctifs dits «mous» par opposition à d'autres glycosaminoglycanes comme l'acide chondroïtine sulfurique présents dans les tissus dits « durs » tels que le cartilage. On le retrouve ainsi en quantités importantes principalement dans la peau.

Le HA est un glycosaminoglycane linéaire non sulfaté composé d'unités répétitives de D-acide glucuronique et de N-acetyl-D-glucosamine (Tammi R., Agren UM., Tuhkanen AL., Tammi M. Hyaluronan metabolism in skin. Progress in Histochemistry & Cytochemistry. 29(2):1-81, 1994).

Dans la peau normale, le HA est synthétisé essentiellement par les fibroblastes dermiques et les kératinocytes épidermiques (Tammi R., déjà cité). Grâce à ses résidus portant une charge négative, le HA joue le rôle d'une pompe à eau permettant de maintenir l'élasticité de la peau. Le HA a un rôle principal dans le contrôle de la diffusion des aliments, des hormones, des vitamines et des sels inorganiques du tissu conjonctif et dans le nettoyage des déchets métaboliques pouvant induire des réactions inflammatoires. Avec l'âge, la quantité de HA et son degré de polymérisation diminuent, résultant en une diminution de la quantité d'eau retenue dans le tissu conjonctif La peau subit alors un processus de vieillissement qui aboutit à une augmentation de la fibrose et à une baisse de la teneur en fibres élastiques. Dans la peau humaine normale, le HA existe sous forme d'un polymère de haut poids moléculaire (600 000 - 1 000 000 Da). La dégradation physiologique du HA dans la peau se fait par (i) l'internalisation par les kératinocytes et (ii) la fragmentation intracellulaire en fragments de taille intermédiaire par les hyaluronidases (60 000-300 000 Da). Le HA fragmenté est relâché par les kératinocytes, passe la membrane basale et est libéré directement dans les vaisseaux lymphatiques (Tammi R. et al., déjà cité).

Le rôle de l'acide hyaluronique (HA) en dermatologie est connu dans de nombreux domaines dont celui de la cicatrisation, et de l'hydratation. L'acide hyaluronique agit le plus souvent par ses interactions avec des protéines de liaison et surtout avec son récepteur transmembranaire CD44 (Tool B.P. 2001, Sem. Cell. Devel. Biol. 12:79-87 ; Liao Y-H., Stuart A.J., Drug Delivery, 12:327-342, 2005). L'activation de ce récepteur se traduit par un rôle de morphogenèse, dans la multiplication et la prolifération cellulaire, l'angiogenèse et la migration cellulaire (G. Weindl, M. Schaller, Skin Pharm. Physiol. 2004, 17:207-213). Les données de la littérature suggèrent que ces différentes actions seraient fonction de l'environnement de la cellule, du poids moléculaire de l'acide hyaluronique et de sa concentration. Par exemple les hauts poids moléculaires inhiberaient l'angiogenèse, alors que les oligosaccharides la stimuleraient.

La demande de brevet japonaise JP-11279042 met en évidence en topique une action de fragments d'acide hyaluronique (HAF) sulfatés (de masse moléculaire comprise entre 1 et 50 kDa) qui permettraient de maintenir l'élasticité de la peau et d'éviter la kératinisation.

Une étude récente réalisée sur des fragments de HA (de masse moléculaire entre 50 et 750 kDa) en topique, met en évidence une relance de la synthèse de l'acide hyaluronique par les kératinocytes (demandes de brevets FR 2 865 651, WO 2005/082327). Cette activité prolifératrice serait induite par des HAF de poids moléculaires précis, activité qui n'apparaîtrait pas sur des poids moléculaires supérieurs ou inférieurs.

Dans le domaine de l'esthétique, de nombreux produits-injectables à base d'acide hyaluronique existent. En mésothérapie, des solutions de vitamines, d'anti-oxydant, de sels minéraux ou d'acide hyaluronique sont utilisées. Les vitamines sont présentes pour stimuler et entretenir le métabolisme cellulaire, et ainsi relancer la production de collagène, les anti-oxydants luttent contre le vieillissement, les sels minéraux seraient indispensables aux activités enzymatiques cellulaires. Quant à l'acide hyaluronique, il permet de maintenir le volume et l'hydratation de la peau, et de créer un écran protecteur face aux dégradations par les radicaux libres (H. Trommer, S. Wartewig, Int. Journ. Of Pharm. 254(2003) 223-234). A des concentrations suffisantes, l'acide hyaluronique créerait un environnement optimal pour la prolifération cellulaire, la néo-synthèse de collagène.

Le stress oxydatif générateur de radicaux libres au niveau du derme et de l'épiderme est responsable du vieillissement cutané et de l'apparition des rides, ridules et de l'affaissement des tissus.

En outre, il a aussi été démontré que les radicaux libres sont aussi responsables de la dépolymérisation de l'acide hyaluronique *in situ,* ce qui contribue d'autant plus au relâchement des tissus et du vieillissement prématuré de ceux-ci (Mendoza G., et al, Carbohydrate Research 342, 2007, 96-102).

La matrice extra cellulaire (MEC) est une structure dynamique ayant un rôle structurel et régulateur pour les tissus. La MEC est constituée de fibres de collagène et d'élastine et aussi de substance fondamentale (principalement eau, sels minéraux et protéoglycanes). Cette matrice confère à la peau sa turgescence et ses propriétés mécaniques de fermeté, élasticité et tonicité.

Les macromolécules de collagène sont des protéines fibreuses formées de trois chaînes polypeptidiques reliées par des liaisons covalentes et hydrogènes. On dénombre 19 types de collagènes dont la moitié dans la peau. La majorité des collagènes dermiques appartiennent aux collagènes fibrillaires de type I, III et V.

Chez le jeune adulte, la composition du derme est de 80 % de collagène de type I et 20 % de collagène de type III ; cependant ce rapport se modifie avec l'âge suite aux phénomènes de vieillissement.

Les élastines sont des protéines organisées en fibres au sein du derme et apportent à la peau ses propriétés d'élasticité et de souplesse. Ces élastines sont riches en acides aminés à caractère hydrophobe.

La dégradation de la MEC intervient au cours de certains processus physiologiques tels que la cicatrisation, le développement embryonnaire ou l'angiogénèse mais aussi lors de situations pathologiques telles que arthrite, arthrose ou athérosclérose voire lors des étapes de progression tumorale avec la formation de métastases (Fisher et al., 1997, N. England J. Med., 337, 1419-28; Shapiro, 1998, Current Oponions in Cell Biology, 10, 602-608).

Les composants de la MEC sont principalement dégradés par des enzymes de type endopeptidases appelées métalloprotéinases matricielles ou MMPs. Ces MMPs participent activement au processus de cicatrisation mais contribuent aussi au relâchement cutané et à l'apparition des rides qui sont les premiers signes du vieillissement cutané. La famille des MMPs comprend environ 22 enzymes qui se distinguent par la spécificité vis-à-vis du substrat qu'elles dégradent.

La MMP-1, ou collagénase interstitielle, dégrade la triple hélice des collagènes fibrillaires de type III majoritairement mais aussi les collagènes I, II, VII, VIII et X.

La MMP-3 dégrade quant à elle les glycoprotéines telles que la fibronectine et la laminine, certains protéoglycanes, l'élastine, la gélatine et les collagènes IV et V. Ces deux MMPs sont exprimées à la fois par les kératinocytes et les fibroblastes.

Dans le domaine du comblement des rides, des gels de HA réticulé chimiquement sont injectés en intradermique pour combler la dépression creusée par la ride. La réticulation permet d'augmenter la rémanence du produit à l'intérieur du derme. Ainsi, si le produit est injecté correctement et selon le profil génétique de chacun, le produit permet un comblement en 4 à 6 voir 8 mois. Il est ensuite totalement résorbé dans le derme.

De tels gels à base de HA ou HA réticulé permettent une réduction de la ride par un effet mécanique de comblement de la dépression cutanée résultant de cette ride. Ces produits ne sont effectivement dotés que de cet effet mécanique et ne contribuent nullement à un quelconque traitement tant préventif que curatif vis-à-vis du vieillissement cutané et de la dégradation de la MEC, essentielle au maintien des propriétés mécaniques de la peau telle son élasticité et sa fermeté. De tels implants, s'ils permettent un effacement des rides ou ridules, procurent un effet limité dans le temps et ne masquent que partiellement les effets du vieillissement intrinsèque de la peau au niveau de ses structures de maintien représentées par la MEC.

Jusqu'à aujourd'hui, plusieurs produits ont été utilisés dans cette même application. Les gels ou huiles de silicones sont faciles à mettre en oeuvre, mais présentent l'inconvénient de migrer dans les tissus situés juste sous les points d'injection. Des phénomènes d'inflammation chroniques ou de réactions allergiques sont ainsi apparus. De plus la silicone n'est pas biodégradable, et se retrouve dans certains organes tels que le foie. Différentes suspensions de particules polymériques ont aussi été proposées, mais la plupart ont provoqué des réactions de rejets, des infections, ou des inflammations. Enfin des suspensions de collagène ont été mises en oeuvre au cours des dernières années. Toutefois le collagène est résorbé relativement rapidement (entre 1 et 3 mois), et provoque certaines réactions allergiques dues à sa provenance, généralement d'origine bovine ou porcine.

Il subsiste donc le besoin de disposer d'implants injectables capables de réaliser une action de comblement de la ride mais aussi de revitaliser le derme et l'épiderme et de contribuer à limiter les processus de sénescence cellulaire accompagnant le vieillissement cutané tout en ne présentant pas les inconvénients évoqués ci-dessus, ou de manière beaucoup moins prononcée, le tout accompagné d'une simplicité et d'un confort accru dans la mise en oeuvre.

La présente invention concerne donc un implant injectable par voie sous-cutanée ou intradermique sous la forme d'un hydrogel monophasique, **caractérisé en ce qu**'il comprend en poids de 0,5 % à 5 %, de préférence 0,5 % à 4 %, plus préférentiellement 2 % d'acide hyaluronique, et dans lequel :
- 50 % à 95 %, de préférence 60 % à 90 %, plus préférentiellement encore 85 % en poids de l'acide hyaluronique est sous forme de gel réticulé ;
- 5 % à 50 %, de préférence 10 % à 30 %, plus préférentiellement encore 15 % en poids de l'acide hyaluronique est sous forme libre, ou sous la forme d'un de ses sels physiologiquement acceptables, d'une masse moléculaire comprise entre 500 et 2800 kDa, de préférence entre 750 et 2600 kDa, plus préférentiellement entre 800 et 2500 kDa, plus préférentiellement encore entre 1000 et 1500 kDa,
dans un fluide vecteur physiologiquement acceptable, le rapport entre le poids du gel d'acide hyaluronique réticulé et le poids de l'acide hyaluronique libre étant compris entre 1:1 et 1:0,05.

Dans une forme particulière de l'invention, l'implant pourra comprendre environ 80 % d'acide hyaluronique réticulé et environ 20 % d'acide hyaluronique libre, ceci principalement dans le cadre d'une application de l'implant selon l'invention au traitement des rides fines. Dans le cas du traitement des rides profondes, la quantité d'acide hyaluronique réticulé sera préférentiellement d'environ 85 % et la quantité d'acide hyaluronique libre d'environ 15 %.

Dans le cadre de la présente invention, par acide hyaluronique ou HA on entend un glycosaminoglycane linéaire non sulfaté composé d'unités répétitives d'acide D-glucuronique et de N-acétyl-D-glucosamine.

On désigne par hydrogel monophasique un hydrogel sous la forme d'une seule phase homogène.

On désigne par sel d'acide hyaluronique physiologiquement acceptable notamment les sels de sodium et de potassium ainsi que leurs mélanges. Avantageusement, le sel est le sel de sodium.

Avantageusement, le gel constitué d'acide hyaluronique réticulé selon l'invention à une viscosité comprise entre 200 et 2000 Pa.s, plus particulièrement entre 500 et 1800 Pa.s, plus particulièrement encore entre 1000 et 1800 Pa.s.

Dans le cas d'un implant destiné au traitement des rides profondes, c'est-à-dire comprenant 85 % d'acide hyaluronique réticulé, la viscosité de celui-ci s'établit aux environs de 1000 à 1500 Pa.s, particulièrement environ 1200 Pa.s. Pour ce qui est d'un implant principalement destiné aux rides légères, donc moins chargé en acide hyaluronique réticulé, c'est-à-dire environ 80 % d'acide hyaluronique réticulé, la viscosité d'un tel implant s'établit aux alentours de 200 à 500 Pa.s, plus s particulièrement aux environs de 350 Pa.s.

Dans la présente description, les viscosités indiquées correspondent à des valeurs mesurées pour un taux de cisaillement de 0,01 s⁻¹.

Avantageusement, l'acide hyaluronique réticulé constituant le gel selon l'invention a une masse moléculaire comprise entre 1000 et 6000 kDa, plus avantageusement comprise entre 1000 et 4000 kDa.

Selon un aspect avantageux de l'invention, l'implant injectable contient en outre du sulfate de chondroïtine.

Avantageusement, la quantité de sulfate de chondroïtine représente en poids entre 0,05 % et 5 % du poids total.

Avantageusement, le sulfate de chondroïtine a une masse moléculaire comprise entre 2 et 80 kDa, plus avantageusement entre 20 et 50 kDa.

L'implant selon l'invention peut contenir divers additifs habituels. On citera à titre d'exemple les colorants, les pigments colorants, les huiles végétales, les épaississants, les modificateurs de pH, les ajusteurs de l'osmolarité.

L'acide hyaluronique libre, ou l'un de ses sels physiologiquement acceptables, est avantageusement réparti de façon homogène à l'intérieur du gel d'acide hyaluronique réticulé.

Le fluide vecteur est avantageusement un tampon isotonique stérile apyrogène.

L'implant injectable selon l'invention contient en outre avantageusement au moins un autre principe actif utilisé en dermo-cosmétique.

Avantageusement, le principe actif dermo-cosmétique est choisi parmi les vitamines, les anti-oxydants, les sels minéraux, les antiseptiques et le sulfate de chondroïtine.

Dans un aspect particulier de l'invention, l'implant injectable contient du mannitol qui exerce un effet anti-oxydant au niveau du derme et contribue à préserver le HA d'une dépolymérisation induite par les radicaux libres générés au sein du derme par le stress oxydatif. En effet l'association du mannitol avec le HA a été décrite comme améliorant la protection contre les dommages induits par les radicaux libres (Belda et al., 2005, J. Cataract Refract. Surg., 31:1213-1218). C'est ainsi un aspect de la présente invention que de fournir un implant injectable tel que défini ci-dessus et contenant un mannitol en tant qu'anti-oxydant.

Un autre aspect de la présente invention réside dans l'utilisation du mannitol au sein d'un implant injectable selon la présente invention afin de protéger le derme des radicaux libres et/ou de limiter la dépolymérisation de l'acide hyaluronique contenu.

L'invention a donc également pour objet l'utilisation d'acide hyaluronique sous forme libre, ou sous la forme d'un de ses sels physiologiquement acceptables, d'une masse moléculaire comprise entre 500 et 2800 kDa, de préférence entre 750 et 2600 kDa, plus préférentiellement entre 800 et 2500 kDa, en présence d'un anti-oxydant, tel que le mannitol, plus préférentiellement encore entre 1000 et 1500 kDa pour la fabrication d'un implant destiné à protéger le derme des radicaux libres et/ou à limiter la dépolymérisation de l'acide hyaluronique du derme et/ou à prévenir le vieillissement cutané.

L'implant selon la présente invention est injecté dans le derme superficiel, moyen ou profond.

Il présente l'avantage de pouvoir, dès l'injection, provoquer une relance fibroblastique en favorisant la prolifération cellulaire et la néo-synthèse de collagène. L'activation des fibroblastes génère alors une modulation des mécanismes impliqués dans le remodelage de la matrice extracellulaire, ce qui se traduit par une revitalisation du derme.

L'implant selon la présente invention présente le double avantage d'obtenir un effet direct et immédiat de réduction de la ride par comblement mécanique de la dépression cutanée et un effet indirect sur un plus long terme de régénération cellulaire *via* une stimulation de la synthèse de collagène et une régulation des MMPs.

L'acide hyaluronique réticulé contribue directement à l'effet mécanique de comblement et permet de par sa nature réticulée d'obtenir un tel effet de manière durable sur des périodes plus longues que le HA non réticulé.

En outre, le HA libre contenu dans l'implant selon l'invention inhibe la surexpression des MMP-1 ainsi que la surexpression du collagène III. De part cette action régulatrice sur les MMP-1, il contribue à la limitation de la déstructuration et de la destruction de la MEC, structure essentielle au maintien des propriétés mécaniques de fermeté et d'élasticité de la peau.

Il a été démontré par ailleurs que le ratio collagène III/collagène I augmentait au cours du vieillissement cutané (Weber et al, 1984, J. Invest. Dermatol, 82, 156-60). Sachant que dans le cadre de la présente invention, il a été constaté que la fraction de HA libre telle qu'utilisée dans l'implant selon l'invention inhibe l'expression du collagène de type III sans affecter celle du collagène de type I, on peut raisonnablement penser que l'implant selon l'invention est susceptible de rétablir le ratio collagène III/collagène I tel que mesuré au sein de tissus jeunes.

C'est ainsi un objet de la présente invention que d'utiliser un implant selon la présente invention pour la fabrication d'un médicament destiné à maintenir et/ou rétablir le ratio collagène III/collagène I tel que mesuré dans les tissus jeunes.

L'association du HA libre de masse moléculaire comprise entre 500 et 2800 kDa avec le gel de HA réticulé a pour conséquence l'hydratation et le maintien du volume de la peau, de façon immédiate. De plus elle induit une relance fibroblastique du derme, principalement due à la présence du HA libre, et cette relance fibroblastique est prolongée dans le temps au fur et à mesure de la dégradation *in vivo* du gel de HA réticulé.

L'implant selon la présente invention peut se représenter comme un maillage tridimensionnel constitué par le HA réticulé comprenant en son sein des molécules de HA libres susceptibles d'induire une stimulation des fibroblastes, d'inhiber la dégradation de la MEC par inhibition des MMPs et de réguler la synthèse de collagène en orientant celle-ci vers un état correspondant à celui observé dans les tissus jeunes.

Ces molécules de HA libres sont libérées progressivement à partir de cette matrice tridimensionnelle de HA réticulé tant par diffusion passive que *via* la dégradation temporelle de la matrice de HA réticulé au cours des semaines et mois suivant l'injection. Ce relargage progressif permet cette action de réjuvénation, de stimulation cellulaire in situ *via* ces molécules de HA libres qui sont préservées de la dégradation biologique au sein de ladite matrice tout au long des semaines et peuvent ainsi exercer leur action sur une période plus longue que si elles étaient injectées seules.

C'est ainsi un objet de la présente invention que l'utilisation d'acide hyaluronique sous forme libre, ou sous la forme d'un de ses sels physiologiquement acceptables, d'une masse moléculaire comprise entre 500 et 2800 kDa, de préférence entre 750 et 2600 kDa, plus préférentiellement entre 800 et 2500 kDa, plus préférentiellement encore entre 1000 et 1500 kDa, réparti au sein d'un gel d'acide hyaluronique réticulé, pour la fabrication d'un implant sous cutané destiné au comblement des rides et à la relance cellulaire épidermique et/ou au maintien des propriétés mécaniques de fermeté et d'élasticité de la peau.

Donc l'implant selon l'invention combine l'effet mécanique du gel réticulé, qui gonfle et remodèle la ride, avec l'action biologique du HA libre.

L'invention a également pour objet un kit se présentant sous la forme de seringue contenant un implant injectable tel que décrit précédemment.

L'invention a encore pour objet l'implant tel que décrit précédemment en tant que médicament.

L'invention concerne également l'utilisation d'un implant injectable tel que précédemment décrit pour le comblement des rides, ridules, dépressions cutanées et/ou cicatrices comprenant l'injection sous-cutanée d'un tel implant.

L'implant injectable selon l'invention peut être utilisé pour la préparation d'un médicament destiné à stimuler le métabolisme épidermique et dermique et/ou à relancer l'activité cellulaire épidermique.

L'implant injectable selon l'invention peut être utilisé pour la préparation d'un médicament destiné à stimuler l'activité anti-oxydante du derme et/ou à prévenir le vieillissement cutané.

L'invention concerne également un procédé cosmétique de comblement des rides et/ou ridules, comprenant l'injection d'au moins un implant injectable selon l'invention.

L'invention a également pour objet un procédé de préparation d'un implant injectable tel que décrit précédemment. Il peut être préparé par toute technique connue de l'homme du métier, par exemple par un diépoxy, notamment le butanediol diglycidyle éther (BDDE) ou le 1,2,7,8-diépoxy-octane. Avec une réticulation en milieu basique, la concentration en diépoxy peut varier par exemple entre 5 et 15 % par rapport à l'acide hyaluronique pour une réticulation en bain-marie à 45-55°C pendant 1,5 à 6 h. Le gel réticulé est ensuite purifié par les techniques classiques de l'homme de l'art : différents bains d'eau désionisée, précipitation alcoolique, dialyse, ... pour éliminer les traces de réticulant résiduel. A ce gel purifié, du HA de poids moléculaire adéquat, préalablement hydraté dans un tampon adéquat, sera ajouté. Le produit fini sera enfin dégazé, mis en seringue ou tout autre récipient adéquat et stérilisé par autoclavage.

Un procédé avantageux selon l'invention comprend les étapes suivantes:
1) préparation d'un gel réticulé selon les étapes suivantes :
   - introduction d'acide hyaluronique dans un fluide basique,
   - gonflement, homogénéisation sous agitation lente et réticulation à chaud,
   - neutralisation et gonflement du gel réticulé dans une solution tamponnée à pH d'environ 7 additionnée d'agent isoosmolarisant,
   - élimination du réticulant,
2) préparation d'un gel d'acide hyaluronique libre par:
   - introduction d'acide hyaluronique dans une solution tamponnée aux environs de pH 7, isoosmolaire ;
   - gonflement,
3) mélange du gel réticulé obtenu à l'étape 1) avec le gel d'acide hyaluronique libre obtenu à l'étape 2),
4) dégazage optionnel et éventuellement conditionnement en flacons ou seringues puis stérilisation.

Avantageusement, le pH du gel après stérilisation est d'environ 7 et l'osmolarité aux environs de 250 à 350 mOsm, préférentiellement entre 300 et 320 mOsm.

L'invention va maintenant être illustrée, de manière non limitative, par les exemples suivants.

### Exemple 1 :

Caractérisation de l'activité de HA de différentes masses moléculaires sur:
- des fibroblastes sains;
- des fibroblastes sénescents (par stress oxydatif à H₂O₂).

La MMP-1 (ou collagénase 1) est une collagénase interstitielle qui dégrade la triple hélice des collagènes fibrillaires comme les collagènes I et III. Au niveau cutané, elle est exprimée et sécrétée par les fibroblastes et les kératinocytes. La MMP-1 est impliquée dans le vieillissement. En effet, sa surproduction au cours du vieillissement pourrait être impliquée dans la perte de fermeté et d'élasticité, et l'apparition des rides. Lorsque l'on induit la sénescence avec H₂O₂, on observe chez les fibroblastes une très forte augmentation de la MMP-1. Les principes actifs capables de réduire cette surproduction pourraient donc avoir des propriétés « anti-âge ».

Les fibroblastes sont obtenus à partir de peau provenant de déchets opératoires de sujets jeunes. Les échantillons ont été lavés dans du PBS et de l'éthanol. Des petits morceaux de peau ont été coupés et répartis dans des boîtes de culture et immergés dans un milieu favorable à la prolifération des fibroblastes (DMEM + 10 % SVF). A ce milieu, de la primocine a été ajoutée. Celle-ci est antibiotique, antifongique et antimicoplasme. Les boîtes de cultures sont mises à incuber.

Pour induire la sénescence, les fibroblastes sont ensemencés dans des boîtes de cultures. 24 heures après, le principe actif à tester est rajouté selon la concentration appropriée, dans du DMEM. Après 24 heures d'incubation, les cellules sont soumises à un stress oxydant. Elles sont incubées pendant 2 heures à 37°C, dans une solution d'H₂O₂ à 75 µM dans du PBS. Il s'agit d'un stress aigu qui induit la sénescence des cellules. Les fibroblastes sont ensuite replacés dans du DMEM complété avec 10 % de SVF. 72 heures après la fin du stress, les fibroblastes sont rentrés en sénescence.

### 1.1. Protocole d'incubation.

Pour les fibroblastes sénescents : les HA sont ajoutés à une concentration finale de 1 µg/ml, ils sont laissés pendant le stress H₂O₂, soit 24 h d'incubation.

Pour les fibroblastes sains : les HA sont incubés également 24 h à 1 µg/ml.

### 1.2. Extraction des ARN totaux à l'aide du kit d'extraction Rneasy (Qiagen) à la fin de l'expérience (J5, c'est-à-dire 72 h post-stress).

### 1.3. Dosage des ARN totaux.

Le dosage qualitatif et quantitatif des ARN totaux est réalisé à l'aide du kit de dosage RNA 6000 Nano LabChip et de l'appareil Bioanalyseur 2100 (Agilent). Il repose sur le principe de la migration électrophorétique des échantillons dans une nanopuce. Le rapport des ARN ribosomaux 28S/18S est calculé; il est informatif pour évaluer l'intégrité des ARN. De plus, la pureté des ARN vis-à-vis de l'ADN génomique est estimée.

### 1.4. Analyse des taux de transcription par PCR en temps réel.

La PCR en temps réel permet de quantifier le niveau d'expression transcriptionnel d'un gène d'intérêt par amplification des ADNc, obtenus par transcription inverse, des ARNm du gène présent dans le lysat cellulaire. Le mélange réactionnel est constitué d'ADNc et du mélange du couple d'amorces d'intérêt avec une solution d'iQ SybrGreen Supermix (Biorad) contenant, entre autres, la Taq Polymérase et un agent intercalant du petit sillon des doubles hélices d'ADN : le Sybr Green (agent intercalant fluorescent). La réaction se fait dans le thermocycleur Icycler IQ (Biorad) ; il s'agit d'une suite de cycles de dénaturation/ hybridation/élongation.

### 1.4.1. Transcription inverse : les ARN totaux sont « retro-transcrits » en ADNc à l'aide du kit Reverse Transcription System (Promega) et de l'appareil Gene Amp PCR System 2400 (Perkin Elmer).

### 1.4.2. Mesure du taux de transcription d'un gène d'intérêt : le niveau d'expression du gène d'intérêt est normalisé par le niveau d'expression de gènes de référence, qui varient peu avec la sénescence. Le niveau d'expression du gène d'intérêt est calculé selon la formule : 2 (CTmin-CT) où CT signifie « cycle threshold ».

Il est normalisé, par rapport à l'expression des trois gènes de référence sélectionnés, selon le calcul : 2 (CTmin-CT) / facteur de normalisation.

### 1.5. Résultats sur l'expression de MMP-1.

Les fibroblastes rendus sénescents par incubation avec H₂O₂ exprimaient 2,14 fois plus la MMP-1 que les fibroblastes normaux (donc la sénescence a bien été induite). En présence de Vitamine E (contrôle positif), la surexpression de MMP-1 par les fibroblastes sénescents était réduite de 40 %.

La surexpression de MMP-1 était réduite de 40 % en présence de HA 450 kDa (10 µg/ml), de 75 % en présence de HA 800 kDa (10 µg/ml), de 71 % en présence de HA 1500 kDa (10 µg/ml), de 83 % en présence de HA 2600 kDa (10 µg/ml).

### 1.6. Résultats sur l'expression du collagène de type I et III.

Sur les fibroblastes rendus sénescents par incubation avec H₂O₂, une inhibition variable de la transcription de collagène de type I a été mesurée; une telle inhibition semblait cependant dépendante des donneurs. Sur un des donneurs pour lequel la transcription du collagène de type I était inhibée de 24 % par le stress à l'H₂O₂, les différents HA de différente taille ne restauraient pas la synthèse de collagène.

Chez les fibroblastes rendus sénescents, une augmentation de +163 % à +304 % de la transcription du collagène de type III a été mesurée. Sur le donneur pour lequel la transcription du collagène de type III est stimulée de +304 % par le stress H₂O₂, les HA libres de différentes tailles inhibent la synthèse de collagène. L'HA de 450 kDa inhibe d'environ 25 %, alors que les HA de masse supérieure à 800 kDa inhibent la transcription d'environ 100 %, pour retrouver des niveaux proches des fibroblastes jeunes.

### 1.7. Conclusion.

En conclusion de cette expérience, il apparaît que les HA exercent une activité anti-MMP-1 (soit anti-oxydante donc ayant potentiellement une activité anti-âge) dans ce modèle de sénescence induite. Les HA de plus haute masse molaire (800, 1500 et 2600 kDa) semblent plus efficaces que le HA de 450kDa.

Dans les conditions expérimentales présentes, les HA de masse moléculaire 450 kDa sont actifs sur la MMP-1. Les HA de masse moléculaire supérieure à 800 kDa, sont plus actifs puisqu'ils inhibent la surexpression de -75 à -83 %.

D'autre part, ces résultats montrent que les différents HA libres n'agissent pas sur la synthèse de collagène de type I.

Enfin, les HA libres inhibent la surexpression de collagène de type III, et cela est particulièrement remarquable pour les HA de masse moléculaire à partir de 800 kDa qui inhibent la surexpression de-118 à -93 %.

Ainsi, de par leur action régulatrice sur les MMP-1, les HA libres contenus dans l'implant selon la présente invention contribuent à limiter la destruction et la déstructuration de la MEC. Par ailleurs, sachant qu'il a été démontré que le rapport collagène III/collagène I augmentait au cours du vieillissement, les HA contenus dans l'implant selon l'invention permettent de rétablir le ratio mesuré dans les tissus jeunes.

### Exemple 2: Préparation d'un implant selon l'invention.

### 2.1. Préparation du HA réticulé.

5 g de hyaluronate de sodium de poids moléculaire de 1,6 MDa ont été ajoutés à de la soude 1 % (35,6 g). Le mélange est laissé à l'homogénéisation discontinue pendant 1 h 30. 315 mg de BDDE ont été ajoutés au mélange HA/NaOH qui a été homogénéisé, fermé puis placé au bain-marie à 50°C pendant 2 h. Le mélange a été neutralisé par l'ajout de 5 g de HCl lN.

Le gel ainsi obtenu a été ajouté à la concentration souhaitée par ajout d'EDI et de sels garantissant l'iso-osmolarité, ainsi qu'un pH neutre stable pour donner un gel à 20 mg/g en HA.

### 2.2. Préparation de l'implant.

A ce gel purifié est ajouté du HA de 1,2 MDa, préalablement hydraté dans du tampon phosphate. Aux 226 g de gel réticulé obtenus, sont ajouté 60 g de gel de HA 1,2 MDa, de concentration 20 mg/g. Les 2 gels sont homogénéisés dans un mélangeur classique à pâles pendant 1 à 2 heures.

Le produit fini peut enfin être dégazé, rempli en seringues et stérilisé par autoclavage vapeur suivant un cycle par exemple de 125°C pendant 7 min, de 127°C pendant 4 min. ou de 130°C pendant 3 min.

Après stérilisation, le pH du produit est de 7,1, l'osmolarité de 320 mOsm, et les caractérisations rhéologiques donnent un module élastique G' de 45 Pa.s à une fréquence de 1 Hz. La concentration finale du gel est dosée à 19,8 mg/g (dosage au carbazole, méthode de la Pharmacopée Européenne), pour une concentration prévue de 20 mg/g.

## Revendications

1. Implant injectable par voie sous-cutanée ou intradermique sous la forme d'un hydrogel monophasique, **caractérisé en ce qu**'il comprend en poids de 0,5 % à 5 %, de préférence 0,5 % à 4 %, plus préférentiellement 2 % d'acide hyaluronique, et dans lequel :
- 50 % à 95 %, de préférence 60 % à 90 %, plus préférentiellement encore 85 % en poids de l'acide hyaluronique est sous forme de gel réticulé;
- 5% à 50 %, de préférence 10 % à 30 %, plus préférentiellement encore 15 % en poids de l'acide hyaluronique est sous forme libre, ou sous la forme d'un de ses sels physiologiquement acceptables, d'une masse moléculaire comprise entre 500 et 2800 kDa, de préférence entre 750 et 2600 kDa, plus préférentiellement entre 800 et 2500 kDa, plus préférentiellement encore entre 1000 et 1500 kDa,
dans un fluide vecteur physiologiquement acceptable, le rapport entre le poids du gel d'acide hyaluronique réticulé et le poids de l'acide hyaluronique libre étant compris entre 1:1 et 1:0,05.

2. Implant injectable selon la revendication 1, **caractérisé en ce que** le gel constitué d'acide hyaluronique réticulé a une viscosité comprise entre 200 et 2000 Pa.s, de préférence entre 1000 et 1800 Pa.s.

3. Implant injectable selon l'une quelconque des revendications précédentes, **caractérisé en ce que** l'acide hyaluronique constituant le gel réticulé a une masse moléculaire comprise entre 1000 et 6000 kDa, de préférence comprise entre 1000 et 4000 kDa.

4. Implant injectable selon l'une quelconque des revendications précédentes, **caractérisé en ce que** le sulfate de chondroïtine a une masse moléculaire comprise entre 2 et 80 kDa, préférentiellement entre 20 et 50 kDa.

5. Implant injectable selon l'une quelconque des revendications précédentes, **caractérisé en ce que** l'acide hyaluronique libre, ou l'un de ses sels physiologiquement acceptables, est réparti de façon homogène à l'intérieur du gel d'acide hyaluronique réticulé.

6. Implant injectable selon l'une quelconque des revendications précédentes, **caractérisé en ce que** le fluide vecteur est un tampon isotonique stérile apyrogène.

7. Implant injectable selon l'une quelconque des revendications précédentes, **caractérisé en ce qu**'il contient en outre au moins un autre principe actif utilisé en dermo-cosmétique.

8. Implant injectable selon la revendication 8, **caractérisé en ce que** le principe actif dermo-cosmétique est choisi parmi les vitamines, les anti-oxydants, et les sels minéraux, les antiseptiques et le sulfate de chondroïtine.

9. Implant injectable selon la revendication 8, **caractérisé en ce que** l'anti-oxydant est le mannitol.

10. Implant injectable selon l'une quelconque des revendications 1 à 9 en tant que médicament.

11. Kit se présentant sous la forme de seringue et contenant un implant injectable selon l'une quelconque des revendications 1 à 9.

12. Utilisation d'acide hyaluronique sous forme libre, ou sous la forme d'un de ses sels physiologiquement acceptables, d'une masse moléculaire comprise entre 500 et 2800 kDa, de préférence entre 750 et 2600 kDa, plus préférentiellement entre 800 et 2500 kDa, plus préférentiellement encore entre 1000 et 1500 kDa, en présence d'un anti-oxydant, tel que le mannitol, pour la fabrication d'un implant destiné à protéger le derme des radicaux libres et/ou à limiter la dépolymérisation de l'acide hyaluronique du derme.

13. Utilisation d'un implant injectable selon l'une quelconque des revendications 1 à 9, ou du kit selon la revendication 11, pour le comblement des rides, ridules, dépressions cutanée et/ou cicatrices comprenant l'injection sous-cutanée d'un tel implant.

14. Utilisation d'un implant injectable selon l'une quelconque des revendications 1 à 9, pour la préparation d'un médicament destiné à stimuler l'activité anti-oxydante du derme et/ou à prévenir le vieillissement cutané.

15. Utilisation d'un implant selon l'une quelconque des revendications 1 à 9, pour la fabrication d'un médicament destiné à maintenir et/ou rétablir le ratio collagène III/collagène I tel que mesuré dans les tissus jeunes.

16. Utilisation d'acide hyaluronique sous forme libre, ou sous la forme d'un de ses sels physiologiquement acceptables, d'une masse moléculaire comprise entre 500 et 2800 kDa, de préférence entre 750 et 2600 kDa, plus préférentiellement entre 800 et 2500 kDa, plus préférentiellement encore entre 1000 et 1500 kDa, réparti au sein d'un gel d'acide hyaluronique réticulé, pour la fabrication d'un implant sous cutané destiné au comblement des rides et à la relance de l'activité cellulaire épidermique et/ou au maintien des propriétés mécaniques de fermeté et d'élasticité de la peau et/ou à la stimulation du métabolisme épidermique et dermique et/ou à stimuler l'activité anti-oxydante du derme et/ou à prévenir le vieillissement cutané.

17. Procédé cosmétique de comblement des rides et/ou ridules, comprenant l'injection d'au moins un implant injectable selon l'une quelconque des revendications 1 à 9.

18. Procédé de préparation d'un implant injectable selon l'une quelconque des revendications 1 à 9, **caractérisé en ce qu**'il comprend les étapes suivantes:
1) préparation d'un gel réticulé selon les étapes suivantes :
- introduction d'acide hyaluronique dans un fluide basique,
- gonflement, homogénéisation sous agitation lente et réticulation à chaud,
- neutralisation et gonflement du gel réticulé dans une solution tamponnée à pH d'environ 7 additionnée d'agent isoosmolarisant,
- élimination du réticulant,
2) préparation d'un gel d'acide hyaluronique libre par:
- introduction d'acide hyaluronique dans une solution tamponnée aux environs de pH 7, isoosmolaire ;
- gonflement,
3) mélange du gel réticulé obtenu à l'étape 1) avec le gel d'acide hyaluronique libre obtenu à l'étape 2),
4) dégazage optionnel et éventuellement conditionnement en flacons ou seringues puis stérilisation.
